# EUROPEAN PATENT APPLICATION

(11) **EP 2 947 451 A1**
(43) Date of publication of application: **25.11.2015**
(21) Application number: 13871798.8
(22) Date of filing: 03.12.2013
(51) Int. Cl.: G01N 27/02, G01N 27/22, G01N 33/48, G01N 33/86

(54) **ELECTRICAL CHARACTERISTIC MEASUREMENT DEVICE**

(30) Priority: 18.01.2013 JP 2013007701
(71) Applicant: Sony Corporation, Tokyo 108-0075 (JP)
(72) Inventor: HAYASHI, Yoshihito, Tokyo 108-0075 (JP); KATSUMOTO, Yoichi, Tokyo 108-0075 (JP); BRUN, Marcaurele, Tokyo 108-0075 (JP); TOYOIZUMI, Teppei, Tokyo 108-0075 (JP)
(74) Representative: D Young & Co LLP
(86) International application number: PCT/JP2013/082452
(87) International publication number: WO 2014/112227

(57) **Abstract**

There is provided an electrical characteristic measuring device including a rotating mechanism that rotates a sample container to be filled with blood to be measured, to any angle, and a measurement unit that applies a voltage between a pair of electrodes installed in the sample container, and chronologically measures electrical characteristics of the blood.

## Description

### Technical Field

The present technology relates to an electrical characteristic measuring device that measures electrical characteristics of blood. More particularly, the present technology relates to a technology for chronologically measuring electrical characteristics of blood and acquiring information such as coagulability of the blood or the like.

### Background Art

Anti-platelet aggregation agents or anti-coagulant agents are prophylactically administered to patients or healthy persons susceptible to thrombosis. Examples of the patients susceptible to thrombus formation include patients with diabetes, arteriosclerosis, cancer, heart disease and respiratory disease; perioperative patients; and patients taking immunosuppressants. Also, examples of the healthy persons susceptible to thrombus include pregnant women and elderly people. As the anti-platelet aggregation agents, acetylsalicylic acid and the like are used; and as the anti-coagulant agents, warfarin, heparin, activated blood coagulation factor Xa inhibitors, direct thrombin inhibitors, and the like are used.

The prophylactic administration of anti-platelet aggregation agents and anti-coagulant agents against thrombosis has the side effect that an excessively high administered dose increases bleeding risk. In order to obtain a sufficient prophylactic effect while inhibiting this side effect, an administration management becomes important in which blood coagulability of an administered subject is timely evaluated, and the drug and dose to be administered are appropriately selected and determined.

A method for a blood coagulability test for managing drug administration includes the prothrombin time-international normalized ratio (PT-INR), the activated partial thromboplastin time (APTT), and the like. Also, a method for a platelet aggregation test includes adding a substance that induces aggregation of platelet to platelet rich plasma (PRP) obtained by centrifuging blood, and measuring a change in transmitted light levels or absorbance associated with the aggregation to determine good or poor in aggregation capacity.

Recently, there is also proposed a technique of acquiring information on blood coagulation system based on a dielectric constant of blood (See Patent Literatures 1 and 2). For example, in the blood coagulation system analyzers disclosed in Patent Literatures 1 and 2, blood to be analyzed is retained in a container provided with electrodes through which voltage is applied to the blood, and an alternating current is applied to the electrodes thereby to measure a complex dielectric constant. These analyzers analyze the obtained complex dielectric constant spectrum according to a predetermined algorithm thereby to evaluate enhancement or reduction of blood coagulability such as a blood coagulation time.

Also, an example of the sample container used for measuring electrical characteristics of a liquid sample such as blood includes a sample cartridge provided with a narrowed portion between opposing two electrodes in order to inhibit a chemical reaction and interface polarization on a contact surface with the electrodes (See Patent Literature 3).

### Citation List

### Patent Literature

[Patent Literature 1] JP 2010-181400A
[Patent Literature 2] JP 2012-194087A
[Patent Literature 3] JP 2012-52906A

### Summary of Invention

### Technical Problem

However, known blood coagulability tests such as PT-INR and APTT substantially evaluate only bleeding risk associated with reduction in blood coagulability caused by excess administration of anti-coagulant agents, and cannot evaluate thrombus risk associated with enhancement in blood coagulability. Also, the existing platelet aggregation test using PRP may require a centrifugation process. This may cause platelet to be activated during this process, thereby inhibiting accurate test results from being obtained. Furthermore, the operation is complicated.

On the contrary, the method of measuring a dielectric constant of blood disclosed in Patent Literatures 1 to 3 can simply and accurately evaluate information on blood coagulability and the like. However, even in this method, the measurement data may be changed by the influence of blood sedimentation.

Therefore, the present disclosure has a main object to provide an electrical characteristic measuring device that can accurately measure electrical characteristics of blood.

### Solution to Problem

An electrical characteristic measuring device according to the present disclosure includes a rotating mechanism that rotates a sample container to be filled with blood to be measured, to any angle, and a measurement unit that applies a voltage between a pair of electrodes installed in the sample container, and chronologically measures electrical characteristics of the blood.

With regard to the electrical characteristic measuring device, the rotating mechanism may intermittently rotate the sample container.

In addition, the rotating mechanism can alternate between forward rotation and reverse rotation.

On the other hand, the measurement unit can apply an alternating voltage between the pair of electrodes, and measure one or both of impedance and a dielectric constant of the blood.

The sample container may include a tubular container body made of insulating material, a first electrode that is made of conductive material and blocks one end of the container body, and a second electrode that is made of conductive material and is arranged so as to be in contact with the blood.

In this case, the other end of the container body is blocked by the second electrode, and a space constituted by the first electrode, the second electrode and the container body is filled with the blood.

### In addition, the second electrode may be provided with a blood injection hole.

Moreover, the sample container can be made of conductive material and include a stopper that is fitted into the blood injection hole.

Here, the stopper can be a screw stopper.

In addition, the stopper may be provided with a vent.

The sample container can be arranged in a manner that a rotation axis of the sample container is parallel to or forms a tilt angle of 45° or less with respect to an installation surface.

In addition, the electrical characteristic measuring device may include an analysis unit that analyzes an action of a blood coagulation system on the basis of the electrical characteristics of the blood measured in the measurement unit.

### Advantageous Effects of Invention

According to the present disclosure, the influence of blood sedimentation can be reduced, thereby enabling electrical characteristics of blood to be accurately measured.

### Brief Description of Drawings

[FIG. 1] FIG. 1 is a diagram illustrating a schematic configuration of an electrical characteristic measuring device according to a first embodiment of the present disclosure.
[FIG. 2] FIG. 2 is an exploded perspective view illustrating a configuration example of a sample container 2 illustrated in FIG. 1.
[FIG. 3] A, B, C and D are a plan view, a side view, a bottom plan view and a cross-sectional view, respectively, of the sample container 2 illustrated in FIG. 2.
[FIG. 4] FIG. 4 is a diagram illustrating a configuration example of a stopper 24 provided with a vent, and A, B, C and D are a plan view, a side view, a bottom plan view and a cross-sectional view along a-a line indicated in C, respectively.
[FIG. 5] FIG. 5 is a diagram illustrating a configuration example of a container holder 33 illustrated in FIG. 1.
[FIG. 6] FIG. 6 is a diagram illustrating an arrangement example of a sample container in an electrical characteristic measuring device according to a second embodiment of the present disclosure.
[FIG. 7] FIG. 7 is a dielectric spectrum of blood in which elevated blood sedimentation is not observed.
[FIG. 8] FIG. 8 is data at 760 kHz in the dielectric spectrum illustrated in FIG. 7.
[FIG. 9] FIG. 9 is a dielectric spectrum of blood in which blood sedimentation is significant.
[FIG. 10] FIG. 10 is data at 760 kHz in the dielectric spectrum illustrated in FIG. 9.
[FIG. 11] FIG. 11 is data at 10.7 MHz in the dielectric spectrum illustrated in FIG. 9.
[FIG. 12] FIG. 12 is a dielectric spectrum measured while rotating the blood in which blood sedimentation is significant.
[FIG. 13] FIG. 13 is data at 760 kHz in the dielectric spectrum illustrated in FIG. 12.
[FIG. 14] FIG. 14 is data at 10.7 MHz in the dielectric spectrum illustrated in FIG. 12.

### Description of Embodiments

Hereinafter, embodiments for carrying out the present disclosure will be described in detail with reference to the accompanying drawings. It is noted that the present disclosure is not limited to the embodiments described blow. Description will be provided in the following order.

### 1. First Embodiment

(Example of electrical characteristic measuring device including sample container rotating mechanism)

### 2. Second Embodiment

(Example of electrical characteristic measuring device including sample container attached in tilted manner)

### <1. First Embodiment>

First, an electrical characteristic measuring device according to a first embodiment of the present disclosure will be described. As described above, measurement results of electrical characteristics of blood can be influenced by blood sedimentation. Also, it is reported that a dielectric constant of uncoagulated blood is changed by blood sedimentation (K. Asami, T. Hanai, Colloid and Polymer Science, vol. 270, 1992, p. 78-84). However, it is not known what influence the blood sedimentation has in a blood coagulation test performed using a dielectric constant, and it has not been studied how such influence can be eliminated.

For this reason, in order to perform more accurate measurement in an electrical characteristic measuring device for blood, such as a dielectric coagulometer, it may be necessary to employ a method being free from the influence of blood sedimentation, or to isolate the influence by blood sedimentation according to an algorithm for correction. However, such techniques have not been established. Furthermore, evaluation of blood sedimentation itself is also useful in the medical field, and it is desired to develop a device that can measure blood coagulation while evaluating blood sedimentation.

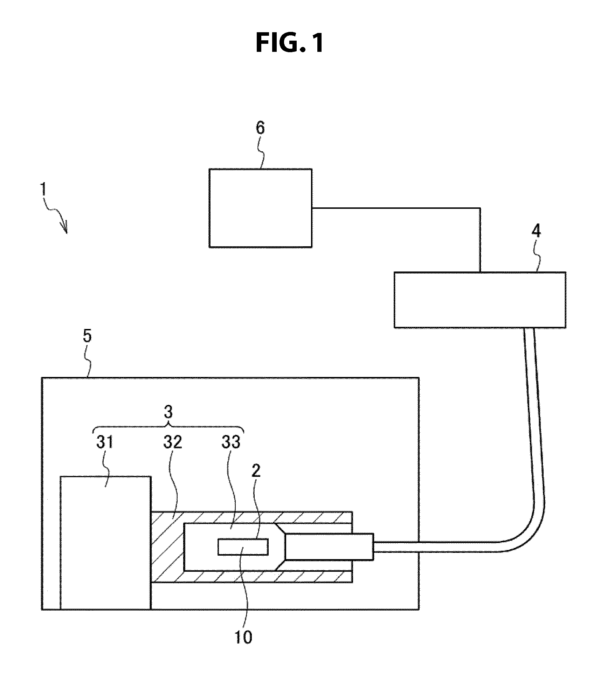

Therefore, the electrical characteristic measuring device according to the present embodiment is configured to rotate a sample container to be filled with blood to be measured, so that the influence by blood sedimentation is inhibited. FIG. 1 is a diagram illustrating a schematic configuration of the electrical characteristic measuring device according to the present embodiment. As illustrated in FIG. 1, an electrical characteristic measuring device 1 according to the present embodiment includes: a rotating mechanism 3 that rotates a sample container 2 to be filled with blood 10 to be measured, to any angle; and a measurement unit 4 that chronologically measures electrical characteristics of the blood 10 filled in the sample container 2.

### [Sample Container 2]

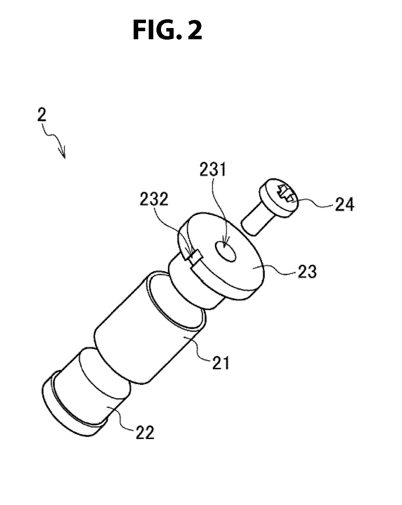

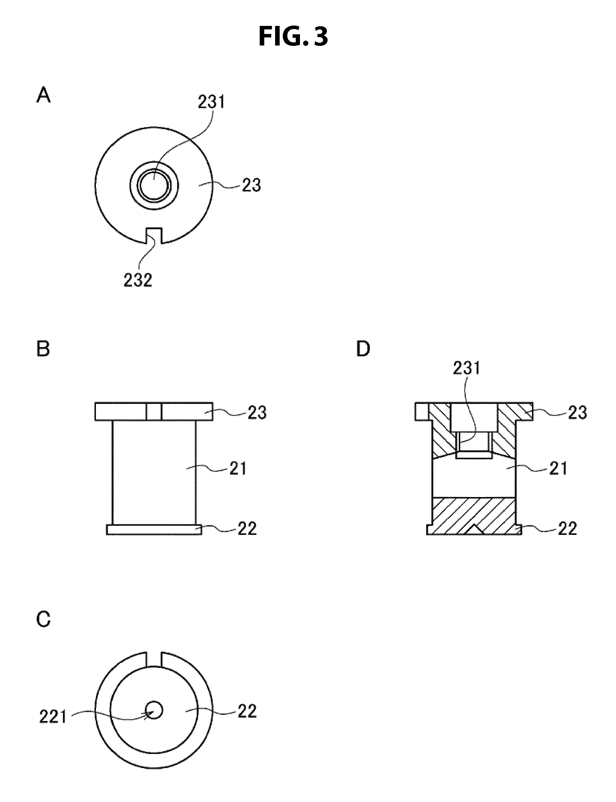

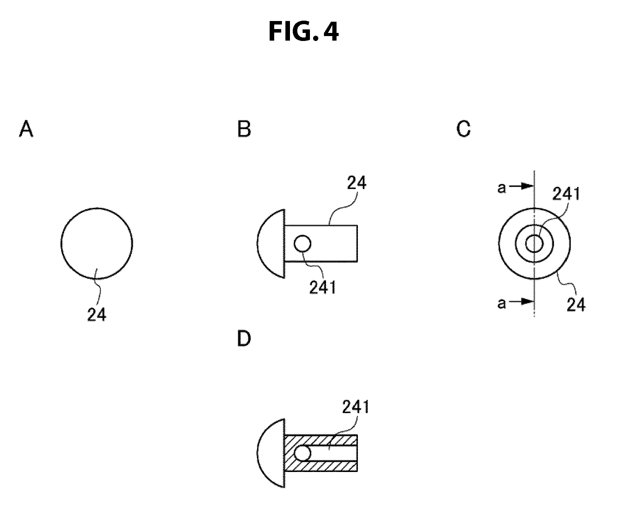

The sample container 2 has a configuration in which the measured blood 10 does not leak, and desirably has a configuration in which impedance mismatches are as small as possible in order to enable high-frequency measurement. FIG. 2 and FIG. 3 are each a diagram illustrating a configuration example of the sample container 2 illustrated in FIG. 1, and FIG. 4 is a diagram illustrating a configuration example of a stopper 24 illustrated in FIG. 2. The sample container 2 may include a pair of or at least two pairs of electrodes, and can contain the blood 10 to be measured. Specifically, the sample container 2 can be configured such that both ends of a tubular container body 21 as illustrated in FIG. 2 and FIG. 3 are blocked with a pair of electrodes 22 and 23.

Here, a material of the container body 21 is not particularly limited as long as it is insulated. Examples thereof may include a hydrophobic and insulating polymer, copolymer and blend polymer such as polypropylene, polymethyl methacrylate, polystyrene and polytetrafluoroethylene. Also, the container body 21 may be obtained by coating the surface of a tubular body made of a predetermined material with these hydrophobic and insulating polymers and the like. It is noted that the shape of the container body 21 may be, other than a round tube illustrated in FIG. 2, a polygonal tube having a cross section of a triangle, a rectangle, or a polygon with more corners.

On the other hand, a material of the electrodes 22 and 23 is not particularly limited as long as it is a conductive material having less influence on blood. Since a stable oxidized thin film is naturally formed in the atmosphere, titanium or titanium alloy, or aluminum or aluminum alloy is suitable. Furthermore, a contact region 221 for electrically connecting with the measurement unit 4 may be installed in the electrode 22 that blocks a bottom end of the container body 21. The contact region 221 can be formed, for example, in a concave shape on the central portion of the outer surface of the electrode 22. This enables stable electrical connection even while the sample container 2 is rotated.

Furthermore, the electrode 23 that blocks an upper end of the container body 21 may include, in the container, a blood injection hole 231 through which the blood 10 to be measured is injected. In this case, the sample container 2 is preferably provided with the stopper 24 that intrudes into and blocks the blood injection hole 231. This can inhibit the blood 10 from leaking out from the blood injection hole 231. A material of the stopper 24 is not particularly limited as long as it is a conductive material having less influence on blood. From a similar reason to for the above-described electrodes 22 and 23, titanium or titanium alloy, or aluminum or aluminum alloy is suitable.

The stopper 24 may also be a screw stopper which can enhance sealing properties. Also, when air remains in the sample container 2, measurement accuracy can deteriorate, and measurement safety can be impaired. Therefore, a vent 241 for releasing remained air may be formed to the stopper 24. The shape of the vent 241 is not particularly limited. An example thereof may include a reversed L shape constituted by a lateral hole and a vertical hole as illustrated in FIG. 4. This enables efficient release of air while inhibiting leakage of liquid.

It is noted that as illustrated in FIG. 3D, a surface on the blood 10 side of the electrode 23 is preferably inclined toward the blood injection hole 231. This allows air remained in the sample container 2 to gather around the blood injection hole 231 when the electrode 22 side is arranged to face downward. Accordingly, the remained air can be surely released.

The angle of this tilted surface installed in the electrode 23 is not particularly limited, and is preferably approximately 140 to 160° when an untilted planar state is assumed to be 180°. As the angle of the tilted surface is closer to a plane (180°), the effect of releasing remained air becomes smaller. On the other hand, when the angle of the tilted surface becomes an acute angle, a difference of the inter-electrode distance becomes larger between the center and the outer edge, causing a formed electric field to become non-uniform. Specifically, the center portion with a long distance between the electrode 22 and the electrode 23 has a weak electric field, and as becoming closer to the outer edge, the distance between the electrode 22 and the electrode 23 increases thereby causing an electric filed to become stronger. As a result, the outer edge having a stronger electric field has higher measurement sensitivity, and in this position, a slight change (blood sedimentation and the like) in the blood 10 have greater influence on measurement results.

Furthermore, a notch 232 may be installed in a flange portion of the electrode 23, so that a screw may be tightened using a jig or the like that engages with this notch 232. This can inhibit idle running of the sample container 2, enables smooth screw tightening, and furthermore, can easily accomplish an automatic screw tightening mechanism by robot actions.

It is noted that the sample container used in the electrical characteristic measuring device according to the present embodiment is not limited to the configuration illustrated in FIGS. 2 to 4, and two or more pairs of electrodes may be installed. Also, for example, the electrodes may be integrated with the container body 21. However, when the sample container 2 is configured to be disposable, the electrode pairs are preferably an independent detachable member.

Furthermore, the electrode 23 may not be provided with the blood injection hole 231, and may also be configured such that, for example, an injection needle is pierced into inner air from the outer surface of the container body 21 to inject the blood 10. In this case, the portion through which the injection needle has penetrated may be blocked with grease or the like in order to seal the container. Still furthermore, there can also be used a sample cartridge disclosed in Patent Literature 3 which has been proposed by the present inventor. However, from the viewpoint of handling properties, it is preferred to use the sample container 2 provided with the blood injection hole 231 in the electrode 23 illustrated in FIGS. 2 and 3.

### [Rotating Mechanism 3]

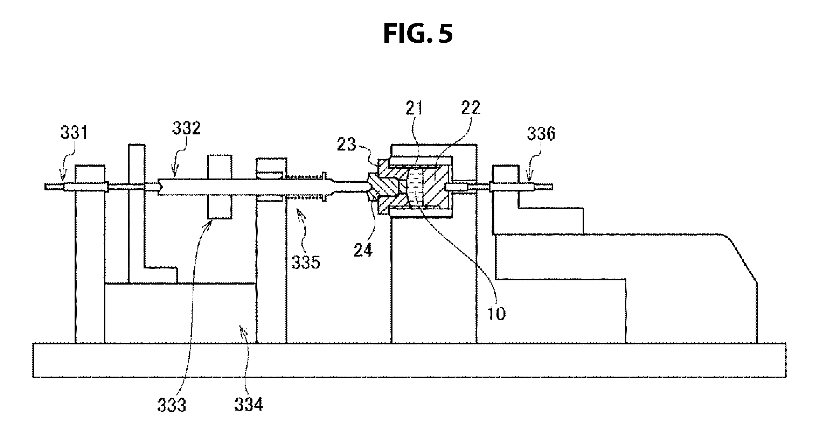

The rotating mechanism 3 includes, for example, a rotor 32 including a container holder 33 attached thereto, and a motor 31 that rotates the rotor 32. FIG. 5 is a diagram illustrating a configuration example of the container holder 33 illustrated in FIG. 1. A method of attaching the sample container 2 to the rotating mechanism 3 is not particularly limited. For example, as illustrated in FIG. 5, the sample container 2 can be arranged such that a rotation axis thereof becomes parallel to an installation surface.

In this case, the electrode 22 is connected with the measurement unit 4 via a sliding contact probe 336 and the like, and the electrode 23 is connected with the measurement unit 4 via a rotation connection member 332 and a sliding contact probe 331. It is noted that the rotation connection member 332 and the sliding contact probe 331 are fixed to a slide mechanism-mounted base 334, thereby enabling their positions to be adjusted.

A gear 333 is attached to the rotation connection member 332, so that the rotation connection member 332 is rotated to any angle when a motor drives the gear 333. Furthermore, a leading end of the rotation connection member 332 has a shape similar to that of a driver being fitted into the groove formed on the head of a screw inserted into the electrode 23. Accordingly, rotation of the rotation connection member 332 causes the whole sample container 2 containing the electrode 23 and the electrode 22 to be rotated.

Furthermore, in order to surely bring the rotation connection member 332 into contact with the electrode 23 and obtain a favorable contact state, a spring 335 that assists a pressing force may also be provided between the rotation connection member 332 and the base 334. Such a configuration can maintain a favorable contact state even during rotation, thereby enabling stable measurement.

### [Measurement Unit 4]

The measurement unit 4 applies a voltage between a pair of electrodes installed in the sample container 2, and chronologically measures electrical characteristics of the blood 10. A configuration of the measurement unit 4 is not particularly limited, and may be appropriately determined according to the electrical characteristics to be measured. For example, when an alternating voltage is applied between a pair of electrodes to measure impedance and dielectric constants of the blood 10, an impedance analyzer and a network analyzer can also be used as the measurement unit 4.

### [Analysis Unit 6]

Furthermore, the electrical characteristic measuring device according to the present embodiment may include an analysis unit 6 that analyzes the action of a blood coagulation system based on the electrical characteristics of the blood 10 measured in the measurement unit 4. The analysis unit 6, for example, determines coagulability and an elevated blood sedimentation level of the blood 10 based on a complex dielectric constant spectrum and its frequency dispersion of the blood 10 measured in the measurement unit 4.

A method for the determination is not particularly limited, and an example thereof includes the method disclosed in Patent Literatures 1 and 2 and proposed by the present inventor. A specific example thereof includes the determination based on a difference in the complex dielectric constant spectrum measured in the coagulation process between the blood added with substances activating or inactivating thrombocytes and the blood without being added with these substances.

### [Operation]

Next, operation of the electrical characteristic measuring device according to the present embodiment will be described. When electrical characteristics of the blood 10 are measured using the electrical characteristic measuring device according to the present embodiment, the sample container 2 is firstly filled with the blood 10 to be measured. At that time, for example, while the electrode 22 is inserted into the container body 21 to block the bottom end, the electrode 23 is not fully inserted, and is inserted to a degree that allows a space larger than an injected volume of the blood 10 to be provided.

In this state, the blood 10 is injected through the blood injection hole 231, and the stopper 24 is fitted into the blood injection hole 231 as necessary. Thereafter, the electrode 23 is pressed into the container body 21. Accordingly, air remained in the container can be easily released without allowing the blood 10 to leak out.

Next, the sample container 2 is attached to the container holder 33, and the electrodes 22 and 23 are connected with the measurement unit 4. Then, the blood 10 is electrically measured while the sample container is rotated to any angle by the rotating mechanism 3. At this time, a rotation pattern of the sample container is not particularly limited. Although continuous rotation may be possible, the sample container is preferably intermittently rotated so that measurement is performed while it is not rotated, from the viewpoint of the stability of electrical connection.

The rotation of the sample container 2 may be unidirectional, or may alternate between forward rotation and reverse rotation. However, the stopper 24 having a screw shape preferably rotates in a direction that the screw is tightened. Also, the rotation angle of the intermittent rotation is most suitably, but not limited to, 180°, and may be other than 180°.

At this time, the measurement unit 4 chronologically measures electrical characteristics such as a complex dielectric constant and a frequency dispersion thereof, starting from when an order to initiate measurement is received or when power is input. For example, in order to measure the complex dielectric constant of the blood 10, the measurement unit 4 applies an alternating voltage between the electrode 22 and the electrode 23, and measures impedance at predetermined intervals. A method of calculating a complex dielectric constant from the measured impedance is not particularly limited, and any known function and relational formula may be used.

Also, the complex dielectric constant can be converted into complex impedance, complex admittance, complex capacitance, complex conductance and the like, through simple conversion of an electrical quantity. Information obtained by analyzing these converted results is equivalent to information obtained by analyzing the complex dielectric constant.

Thereafter, the analysis unit 6 determines coagulability and an elevated blood sedimentation level of the blood 10 as necessary. Also, the determination results in the analysis unit 6 and the measurement results in the measurement unit 4 can be output to a printer (not shown) for printing, and can be output to a display apparatus (not shown) for displaying.

The electrical characteristic measuring device according to the present embodiment chronologically measures electrical characteristics of blood while the sample container is rotated by the rotating mechanism. Therefore, the influence of blood sedimentation can be reduced compared to in existing methods. This enables electrical characteristics of blood to be accurately measured.

Furthermore, the electrical characteristic measuring device according to the present embodiment can evaluate an elevated degree of blood sedimentation without adopting other methods, thereby saving time and labor for conducting a test. A simple evaluation method of the contribution degree of erythrocytes to enhanced coagulation has not existed. However, the use of the electrical characteristic measuring device according to the present embodiment enables such evaluation.

Specifically, centrifugation or the like is used to adjust specimen samples having different hematocrit values, and blood coagulation is dielectrically measured. As the hematocrit value increases, the blood coagulation time becomes shorter. Furthermore, as this decrease rate is higher, the contribution of erythrocytes to enhanced blood coagulation is higher. At this time, for evaluation of the decrease rate, a negative tilt of a liner equation may be simply used, or a fitting parameter obtained by more accurately fitting in a certain function expression may be used.

Also, since the method using centrifugation takes time and labor, a simple evaluation method may also be used in which the contribution of erythrocytes to enhanced blood coagulation is estimated from a difference in the blood coagulation time of the whole blood of a blood specimen used as it is, between when measured while inhibiting blood sedimentation and when measured without inhibiting blood sedimentation. However, in such a case, since the above-described difference is affected by the occurrence level of blood sedimentation, correction may be necessary based on the elevated degree of blood sedimentation.

It is noted that this elevated degree of blood sedimentation can be quantified with a peak of the dielectric constant change by blood sedimentation, which occurs around several kHz to several hundred kHz and is observed in the dielectric blood sedimentation measurement without inhibiting blood sedimentation.

As described above, the electrical characteristic measuring device according to the present embodiment accomplishes a new blood coagulation measurement method that can evaluate risk of thrombosis. Accordingly, even a specimen of a patient having significant elevated blood sedimentation due to various factors such as infections can be accurately evaluated for blood coagulability. In addition, since a patient having high risk of thrombosis is considered to have a certain fundamental disease in many cases, the present disclosure is a technology that is important in clinical applications.

### <2. Second Embodiment>

Next, an electrical characteristic measuring device according to a second embodiment of the present disclosure will be described. In the above-described first embodiment, both ends of the container body are blocked with a pair of electrodes. However, the present disclosure is not limited to this configuration, and one of the pair of electrodes may be installed so as to be in contact with blood as long as the other blocks one end of the container body. That is, the sample container may be an open system in which the upper end is not blocked.

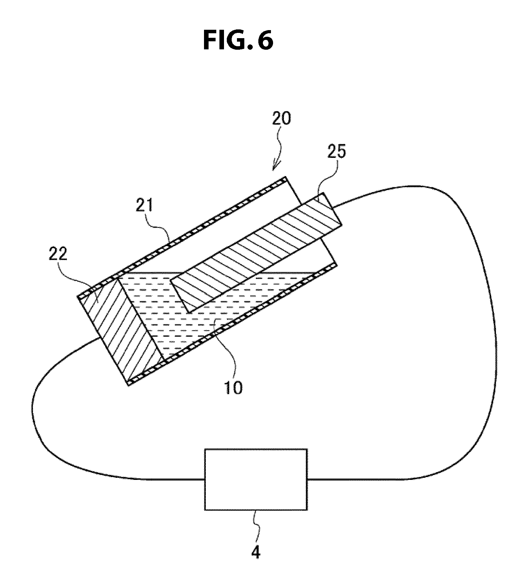

FIG. 6 is a diagram illustrating an arrangement example of the sample container in the electrical characteristic measuring device according to the present embodiment. It is noted that in FIG. 6, components other than the sample container 20 and the measurement unit 4 are omitted for simplification of the diagram. As illustrated in FIG. 6, the sample container 20 being an open system is used in the electrical characteristic measuring device according to the present embodiment.

### [Sample Container 20]

In the sample container 20, a bottom end of the container body 21 is blocked with the electrode 22, while an upper end of the container body 21 is open. Furthermore, an electrode 25 is installed so as to be in contact with the blood 10 to be measured. These electrodes 22 and 25 are each connected with the measurement unit 4 via a cable or the like.

### [Operation]

In the electrical characteristic measuring device according to the present embodiment, the sample container 20 is installed so as to tilt to a degree that the blood 10 does not spill. The angle may be appropriately set depending on an injected amount of the blood 10. For example, the sample container 20 can be installed such that the rotation axis has a tilt angle of 45° or less with respect to an installation surface. Then, electrical characteristics of blood are chronologically measured while the sample container is rotated at any angle by the rotating mechanism, in a similar manner to the above-described first embodiment.

Accordingly, the sample container can have a configuration in which sealing is not necessary while the influence of blood sedimentation is eliminated. As a result, for example, blood can be easily injected, thereby improving workability. It is noted that the configuration, action and effect other than the above of the electrical characteristic measuring device according to the present embodiment are similar to those of the above-described first embodiment.

Additionally, the present technology may also be configured as below.
(1) An electrical characteristic measuring device including:
   a rotating mechanism that rotates a sample container to be filled with blood to be measured, to any angle; and
   a measurement unit that applies a voltage between a pair of electrodes installed in the sample container, and chronologically measures electrical characteristics of the blood.
(2) The electrical characteristic measuring device according to (1),
   wherein the rotating mechanism intermittently rotates the sample container.
(3) The electrical characteristic measuring device according to (1) or (2),
   wherein the rotating mechanism alternates between forward rotation and reverse rotation.
(4) The electrical characteristic measuring device according to any one of (1) to (3),
   wherein the measurement unit applies an alternating voltage between the pair of electrodes, and measures one or both of impedance and a dielectric constant of the blood.
(5) The electrical characteristic measuring device according to any one of (1) to (4),
   wherein the sample container includes
   a tubular container body made of insulating material,
   a first electrode that is made of conductive material and blocks one end of the container body, and
   a second electrode that is made of conductive material and is arranged so as to be in contact with the blood.
(6) The electrical characteristic measuring device according to (5),
   wherein the other end of the container body is blocked by the second electrode, and
   wherein a space constituted by the first electrode, the second electrode and the container body is filled with the blood.
(7) The electrical characteristic measuring device according to (5) or (6),
   wherein the second electrode is provided with a blood injection hole.
(8) The electrical characteristic measuring device according to (7),
   wherein the sample container is made of conductive material and includes a stopper that is fitted into the blood injection hole.
(9) The electrical characteristic measuring device according to claim 8,
   wherein the stopper is a screw stopper.
(10) The electrical characteristic measuring device according to (8),
   wherein the stopper is provided with a vent.
(11) The electrical characteristic measuring device according to any one of (1) to (10),
   wherein the sample container is arranged in a manner that a rotation axis of the sample container is parallel to or forms a tilt angle of 45° or less with respect to an installation surface.
(12) The electrical characteristic measuring device according to any one of (1) to (11), further including:
   an analysis unit that analyzes an action of a blood coagulation system on the basis of the electrical characteristics of the blood measured in the measurement unit.

### [Example]

Hereinafter, effects of the present disclosure will be specifically described. In the present embodiment, the effect of inhibiting blood sedimentation was confirmed using sample blood of healthy persons and patients having significant blood sedimentation.

### (1) Blood Collection

Using a vacuum blood collection tube in which sodium citrate was treated as an anti-coagulant agent, blood was collected from each of healthy persons and patients having significant blood sedimentation to obtain a specimen blood.

### (2) Dielectric Measurement

To the specimen blood that was thermally insulated at 37°C, 0. 25 M aqueous calcium chloride solution was added at a concentration of 85 µL per 1 mL of blood immediately before the start of measurement, to initiate a blood coagulation reaction. Then, measurement was performed for 60 minutes under the conditions of a measurement temperature of 37°C, a measurement frequency range of 40 to 110 MHz, and a measurement interval of one minute.

An apparatus having a configuration illustrated in FIG. 1 was used for the measurement. Specifically, an impedance analyzer (4294A) manufactured by Agilent Technologies Inc. was used as the measurement unit 4. Then, a probe kit (42941A) was connected to the impedance analyzer (4294A), and a rebuilt high-frequency coaxial conversion adapter (a conversion adapter from SMA to APC7) was further connected to a leading end of the probe kit.

One of the electrodes 22 and 23 of the sample container 2 was connected with an inner conductor of the rebuilt coaxial adapter, and the other was contact-connected with an outer conductor of the rebuilt adapter. This enabled broadband measurement containing a high-frequency range. Then, measurement was performed while the sample container 2 was rotated to 180° in forward and reverse directions by motor drive. It is noted that although the probe kit (42941A) was twisted at 180° and straightened back every time the sample container 2 was rotated, no influence was exerted on the measurement.

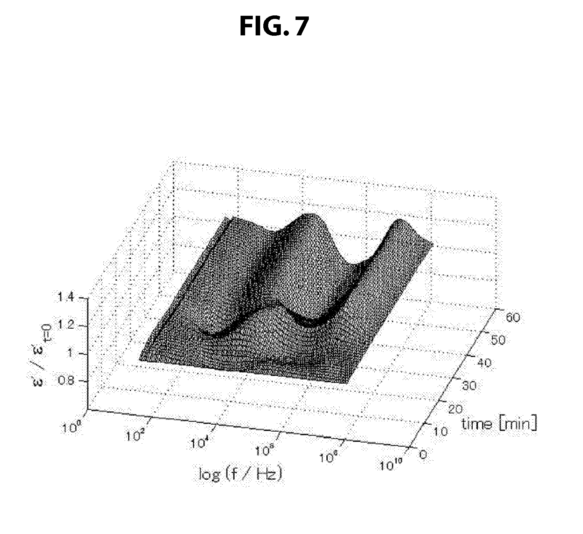

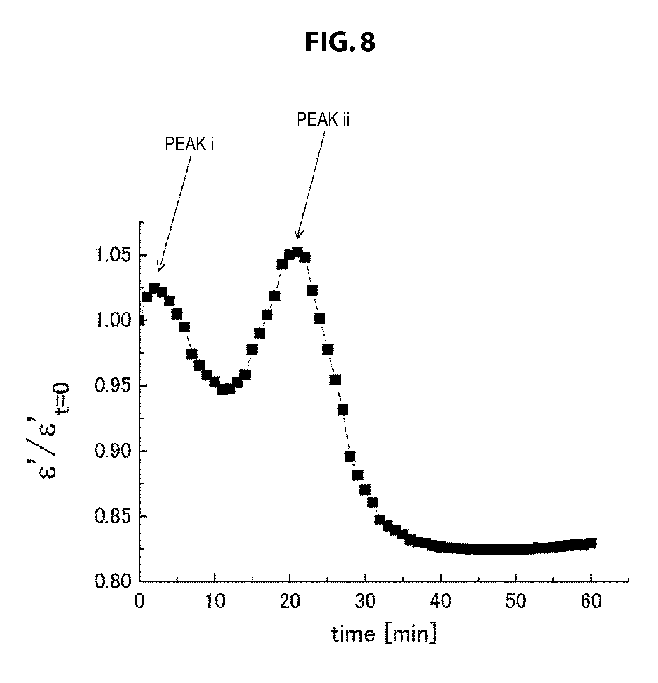

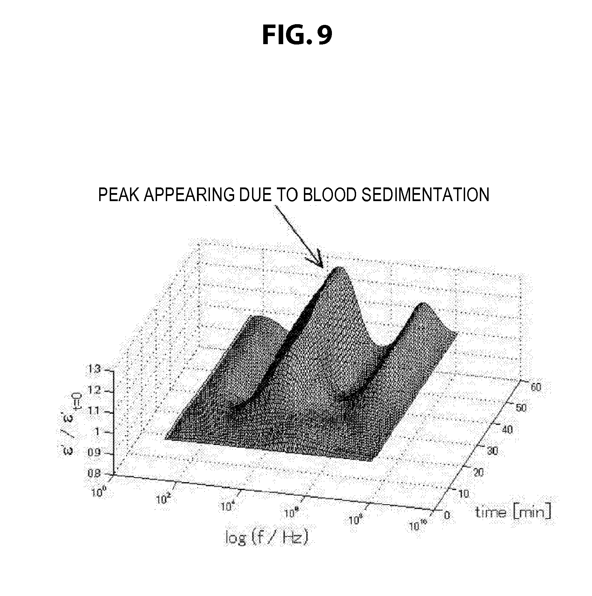

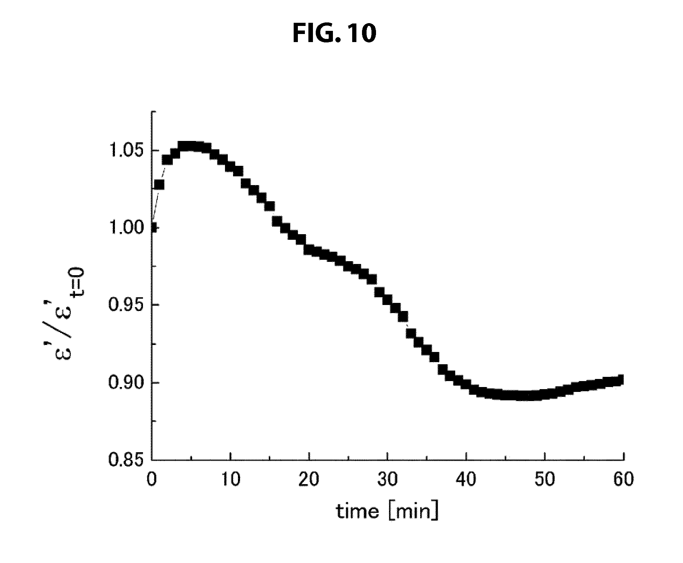

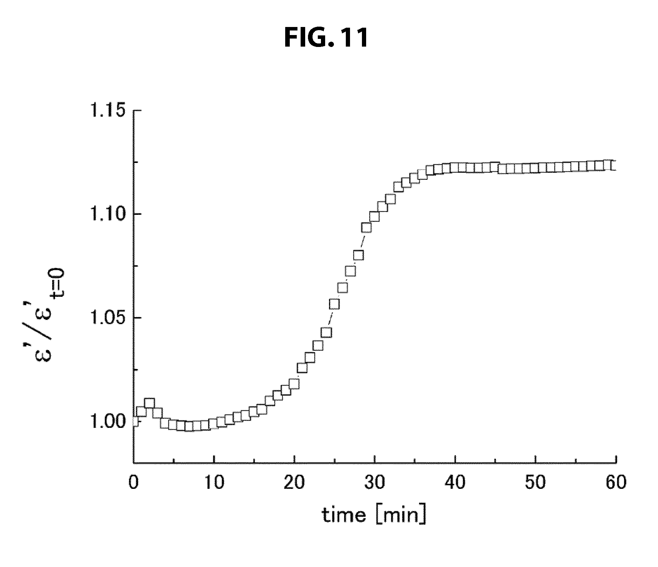

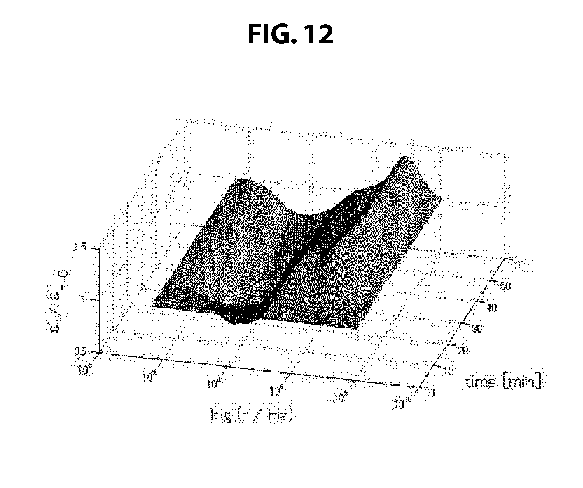

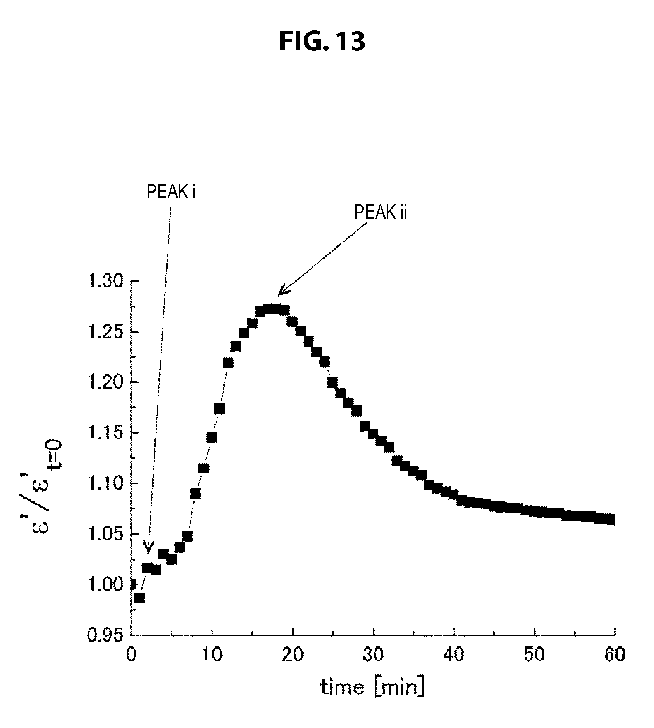

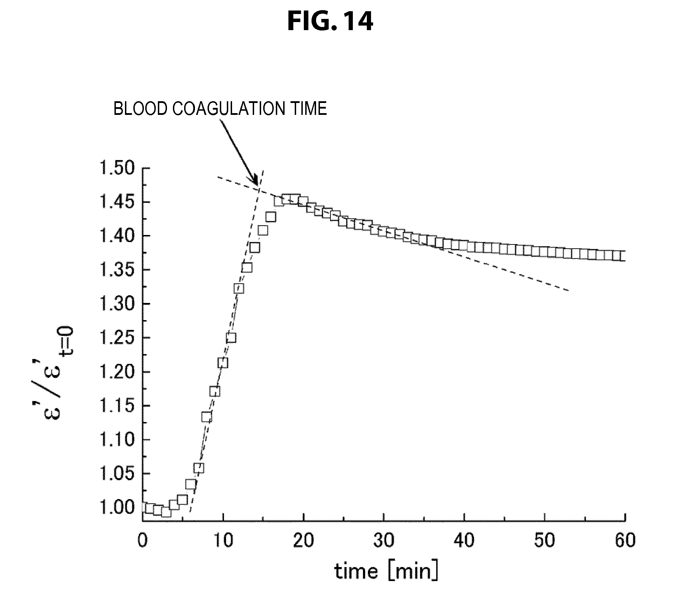

FIG. 7 is a dielectric spectrum of the blood in which elevated blood sedimentation is not observed, and FIG. 8 is the data at 760 kHz. Also, FIG. 9 is a dielectric spectrum of the blood in which elevated blood sedimentation is significant, FIG. 10 is the data at 760 kHz, and FIG. 11 is the data at 10. 7 MHz. Furthermore, FIG. 12 is a dielectric spectrum measured while rotating the blood in which elevated blood sedimentation is significant, FIG. 13 is the data at 760 kHz, and FIG. 14 is the data at 10. 7 MHz.

As illustrated in FIG. 7 and FIG. 8, in the blood in which elevated blood sedimentation is not observed, peak (i) by rouleaux formation of erythrocytes is firstly observed, and thereafter peak (ii) by blood coagulation is obtained. In this case, the time when peak (ii) is provided can be defined as a "blood coagulation time".

On the other hand, as illustrated in FIG. 9, when blood sedimentation is significant, the peak of the change in a dielectric constant by blood sedimentation appears in several kHz to several hundred kHz. Also, as illustrated in FIG. 10, the dielectric constant at 760 kHz decreases after the first peak attributable to rouleaux of erythrocytes is observed, and the second peak related to blood coagulation becomes difficult to distinguish.

On the contrary, FIGS. 11 to 14 are data obtained by measuring a blood sedimentation process while repeating forward rotation and reverse rotation at an angle of 180° every one minute to inhibit blood sedimentation. These data are results of the measurement performed with the same blood specimen as for the data illustrated in FIGS. 9 and 10 on the same experiment day. The comparison between the both data enables the effect of inhibiting the blood sedimentation by the rotation of the sample container 2 to be studied.

Specifically, the peak appearing due to blood sedimentation observed in the range of several kHz to several hundred kHz in the dielectric spectrum illustrated in FIG. 9 disappeared in the dielectric spectrum illustrated in FIG. 12. Also, in the dielectric constant at 760 kHz illustrated in FIG. 13, the peak (i) by rouleaux of erythrocytes was relatively suppressed, and the peak (ii) by blood coagulation was able to be clearly distinguished.

Furthermore, data around 10 MHz can be used to evaluate a blood coagulation time and the like. When comparing the dielectric constant at 10.7 MHz between the result measured while inhibiting blood sedimentation by the rotation of the sample container 2 (FIG. 14) and the result measured without the rotation (FIG. 11), the step-like change by blood coagulation occurs for a shorter time when measured while rotating the sample container 2. That is, the blood coagulation time is shorter when measured while inhibiting blood sedimentation by the rotation of the sample container 2 than when measured without the rotation. This phenomenon was almost commonly observed with minor differences even in other specimens in which blood sedimentation is significant.

Furthermore, the use of these data also enables evaluation of an elevated blood sedimentation degree. As described above, when blood sedimentation occurs, an increase in a dielectric constant is observed in the range of several kHz to several hundred kHz, and the amount of this increase can be used as an index of blood sedimentation. Furthermore, when measurement is performed without adding calcium to a specimen blood, blood coagulation does not occur, thereby enabling only the influence of blood sedimentation to be measured.

The above results clearly show that according to the present disclosure, the influence of blood sedimentation can be reduced, and electrical characteristics of blood can be accurately measured.

### Reference Signs List

- 1: electrical characteristic measuring device
- 2, 20: sample container
- 3: rotating mechanism
- 4: measurement unit
- 5: constant temperature bath
- 6: analysis unit
- 10: blood
- 21: container body
- 22, 23, 25: electrode
- 24: stopper
- 31: motor
- 32: rotor
- 33: container holder
- 221: contact region
- 231: blood injection hole
- 232: notch
- 241: vent
- 331, 336: contact probe
- 332: rotation connection member
- 333: gear
- 334: base
- 335: spring

## Claims

1. An electrical characteristic measuring device comprising:
a rotating mechanism that rotates a sample container to be filled with blood to be measured, to any angle; and
a measurement unit that applies a voltage between a pair of electrodes installed in the sample container, and chronologically measures electrical characteristics of the blood.

2. The electrical characteristic measuring device according to claim 1,
wherein the rotating mechanism intermittently rotates the sample container.

3. The electrical characteristic measuring device according to claim 1,
wherein the rotating mechanism alternates between forward rotation and reverse rotation.

4. The electrical characteristic measuring device according to claim 1,
wherein the measurement unit applies an alternating voltage between the pair of electrodes, and measures one or both of impedance and a dielectric constant of the blood.

5. The electrical characteristic measuring device according to claim 1,
wherein the sample container includes
a tubular container body made of insulating material,
a first electrode that is made of conductive material and blocks one end of the container body, and
a second electrode that is made of conductive material and is arranged so as to be in contact with the blood.

6. The electrical characteristic measuring device according to claim 5,
wherein the other end of the container body is blocked by the second electrode, and
wherein a space constituted by the first electrode, the second electrode and the container body is filled with the blood.

7. The electrical characteristic measuring device according to claim 6,
wherein the second electrode is provided with a blood injection hole.

8. The electrical characteristic measuring device according to claim 7,
wherein the sample container is made of conductive material and includes a stopper that is fitted into the blood injection hole.

9. The electrical characteristic measuring device according to claim 8,
wherein the stopper is a screw stopper.

10. The electrical characteristic measuring device according to claim 8,
wherein the stopper is provided with a vent.

11. The electrical characteristic measuring device according to claim 1,
wherein the sample container is arranged in a manner that a rotation axis of the sample container is parallel to or forms a tilt angle of 45° or less with respect to an installation surface.

12. The electrical characteristic measuring device according to claim 1, further comprising:
an analysis unit that analyzes an action of a blood coagulation system on the basis of the electrical characteristics of the blood measured in the measurement unit.
